# EUROPÄISCHE PATENTSCHRIFT

(11) **EP 4 360 536 B1**
(45) Veröffentlichungstag und Bekanntmachung des Hinweises auf die Patenterteilung: **18.02.2026**
(21) Anmeldenummer: 23150974.6
(22) Anmeldetag: 10.01.2023
(51) Int. Cl.: A61B 3/113

(54) **KOPFGETRAGENE VORRICHTUNG ZUR AUFZEICHNUNG EINER AUGENBEWEGUNG**
HEAD-WORN APPARATUS FOR RECORDING EYE MOVEMENT
DISPOSITIF PORTÉ SUR LA TÊTE POUR ENREGISTRER UN MOUVEMENT OCULAIRE

(30) Priorität: 26.10.2022 EP 22203793
(43) Veröffentlichungstag der Anmeldung: 01.05.2024
(73) Patentinhaber: Zeisberg GmbH, 72555 Metzingen (DE)
(72) Erfinder: Zeisberg, Sven, 72800 Eningen unter Achalm (DE)
(74) Vertreter: Christ, Niko

(56) Entgegenhaltungen:
- EP-A1- 2 851 000
- US-B2- 7 520 614

## Beschreibung

Die vorliegende Erfindung betrifft eine kopfgetragene Vorrichtung zur Aufzeichnung einer Augenbewegung, mit einem Brillenkörper, welcher gemeinsam mit einer Abdeckung eine allseits geschlossene, jedoch über eine Einblicköffnung einsehbare Kammer ausbildet, wobei der Kammer wenigstens ein im sichtbaren Spektrum durchsichtiger Infrarotspiegel zur Reflexion von Infrarotlicht auf wenigstens eine oberhalb der Kammer angeordnete Infrarotkamera zugeordnet ist. Eine derartige Vorrichtung ist bereits aus der US 7,520,614 B2 vorbekannt. Darüber hinaus ist auf die EP 2 851 000 A1 als weiterer Stand der Technik zu verweisen.

Das bereits seit längerem bekannte Verfahren der Video-Okulografie dient der Aufzeichnung von Augenbewegungen und wird in der Medizintechnik für die Gleichgewichtsdiagnostik im Bereich der HNO-Medizin und der Neurologie eingesetzt. Die technische Umsetzung erfolgt durch die Untersuchung mit VideoBrillen, die mithilfe von einer oder zwei Kameras an einer solchen kopfgetragenen Vorrichtung ein Bild des Auges aufzeichnen. Mithilfe geeigneter Bildverarbeitungssoftware wird ein Videostream aufgezeichnet, in dem die Pupille in jedem Einzelbild erkannt und ihre Position über die Zeit verfolgt wird, es wird in diesem Zusammenhang von Eye Tracking gesprochen.

Unter den Video-Okulografie-Verfahren wird zwischen einer lichtausschließenden Anwendung und einer Freisichtanwendung unterschieden. Die lichtausschließende Anwendung arbeitet mit einer Vorrichtung, bei welcher der Proband in eine dunkle Kavität der Vorrichtung hineinblickt, was eine Unterdrückung der Fixationsmöglichkeit des Probanden bezweckt. Um dennoch eine Aufnahme der Augen erstellen zu können, werden in diesen Brillen Infrarotkameras eingesetzt und die Augen mit Infrarot-LEDs beleuchtet. Um die Kameras nicht in den Blickbereich des Probanden montieren zu müssen und dadurch die freie Sicht des Probanden auf ein feststehendes oder bewegtes Blickziel zu erlauben, wird das Bild des Auges mithilfe von Infrarotspiegeln zur Seite oder nach oben umgelenkt.

Die Dauer einer solchen Untersuchung beträgt zwischen 15 und 60 Minuten. Dies erfordert zudem ein Mindestmaß an Tragekomfort und Belüftung, auch, um ein Beschlagen von Kameras, Infrarotspiegeln und Optiken zu verhindern.

Heutzutage handelt es sich bei den meisten Brillen um sogenannte Kombisysteme, die sowohl eine Freisicht- als auch eine lichtausschließende Anwendung erlauben. Der lichtausschließende Betrieb wird dabei durch eine Abdeckung erreicht, welche auf den Brillenkörper aufgesetzt wird. In dieser Anwendung ist es wichtig, dass ein völliger Lichtausschluss erreicht wird, da gerade kleine Spalte vor dem überwiegend dunklen Hintergrund zu scharf erkennbaren Fixationspunkten werden.

Ein solcher völliger Lichtausschluss ist allerdings in der Praxis in den meisten Fällen nicht erreichbar, da die Gesichtsformen der Probanden zu unterschiedlich sind. Eine vollständige Abdichtung, wie etwa bei einer Taucherbrille, ist wenig hilfreich, da dies zu einem schnellen Beschlagen der Optiken führt.

Hinzu kommt, dass sich die Infrarotbeleuchtung in der Pupille spiegelt, was sporadisch zu Beeinträchtigungen des Eye Trackings führt. Da sich die Pupille im Bild bewegt, ändert sich die Position der Spiegelung. Je nach Position wird der Rand der Pupille durch die Spiegelung überdeckt. Die Berechnung der Pupillenposition wird dann fehlerhaft durchgeführt, da hierzu die Mitte der Pupille herangezogen wird.

Bei Verwendung einer Infrarotbeleuchtung wird die Aufzeichnung des Videobildes als Grauwertbild dargestellt. Dies reduziert die Bildinformationen im Vergleich zur Farbdarstellung und erschwert die Bildverarbeitung des Eye Tracking. Während derzeitige Anwendungen häufig nur die X/Y-Position der Pupille aufzeichnen, verlangen neue diagnostische Verfahren die Aufzeichnung der torsionalen Augenbewegung. Diese setzt die Erkennung und Verfolgung kleinster Strukturen im Augenbild, wie etwa Adern in der Iris, etc. voraus.

Vor diesem Hintergrund liegt der vorliegenden Erfindung die Aufgabe zu Grunde, eine kopfgetragene Vorrichtung zur Aufzeichnung einer Augenbewegung zu schaffen, welche die Nachteile des bekannten Standes der Technik im Hinblick auf die Fixation aufgrund unerwünschten Lichteinfalls behebt und die Erfassung einer torsionalen Augenbewegung verbessert.

Gelöst wird diese Aufgabe durch eine kopfgetragene Vorrichtung zur Aufzeichnung einer Augenbewegung gemäß dem unabhängigen Anspruch 1. Sinnvolle Ausgestaltungen können den sich hieran anschließenden, abhängigen Ansprüchen entnommen werden.

Es ist eine kopfgetragene Vorrichtung zur Aufzeichnung einer Augenbewegung vorgesehen, mit einem Brillenkörper, welcher gemeinsam mit einer Abdeckung eine allseits geschlossene, jedoch über eine Einblicköffnung einsehbare Kammer ausbildet, wobei der Kammer wenigstens ein im sichtbaren Spektrum durchsichtiger Infrarotspiegel zur Reflexion von Infrarotlicht auf wenigstens eine oberhalb der Kammer angeordnete Infrarotkamera zugeordnet ist. Eine solche Vorrichtung ist erfindungsgemäß dadurch gekennzeichnet, dass der Brillenkörper und die Abdeckung aus einem trüben und transluzenten Material hergestellt ist und eine der Kammer zugewandte, kantenfreie Innenfläche aufweist.

Eine solche kopfgetragene Vorrichtung zur Aufzeichnung der Augenbewegung umfasst einen Brillenkörper, welcher zusammen mit einer Abdeckung eine allseits geschlossene Kammer ausbildet. Diese Kammer ist jedoch über eine Einblicköffnung einsehbar. Der Brillenkörper und die Abdeckung sind aus einem trüben und gleichzeitig transluzenten Material aufgebaut, so dass zwar Licht in das Innere der Vorrichtung fällt, aber der Proband im Inneren der Vorrichtung keine Fixierpunkte auffindet. Für die Interpretation der Augenbewegung ist für einige diagnostische Verfahren der Ausschluss der Fixation unabdingbar, da die Fixation die durch andere Reize, etwa durch thermische Reize bei der sogenannten kalorischen Prüfung oder Kopfbewegungen bei Lagetests und Lagerungstests, verursachte Augenbewegung unterdrückt.

Um dies zu verhindern ist die dem Auge zugewandte Innenfläche der im Übrigen allerseits geschlossenen Kammer kantenfrei aufgebaut, mithin verrundet und mit möglichst gleichmäßigen Materialdicken für einen gleichmäßigen Lichteinfall versehen. Weil das Material undurchsichtig ist, entsteht beim Hineinsehen in die Kammer eine getrübte visuelle Wahrnehmung. Gleichzeitig kann das Tageslicht mit einem Infrarotlichtanteil von außen in die Kammer eintreten, zunächst am Auge reflektiert werden und von einem Infrarotspiegel auf eine Infrarotkamera reflektiert werden, welche oberhalb der Einblicköffnung auf den Infratotspiegel gerichtet ist, sodass die Augenbewegung aufgezeichnet werden kann. Dies ist von besonderem Vorteil, da in einer Ausführung, in der das Material lichtdurchlässig ist, es nicht nötig ist, eine Infrarotstrahlungsquelle nahe dem Auge am Brillenkörper zu befestigen. Eine solche Infrarotstrahlungsquelle stellt immer ein Risiko da, da die Wärmestrahlung im schlimmsten Fall die Netzhaut beschädigen kann.

Vorzugsweise kann der Brillenkörper und/oder die Abdeckung aus einem Material aus einem weißen Kunststoff oder einem klaren Kunststoff, letzterer vorzugsweise mit angerauter Oberfläche hergestellt sein. Kunststoff zeichnet sich durch seine Verformbarkeit, Härte, Elastizität, Bruchfestigkeit, Temperatur-, Wärmeformbeständigkeit und chemische Beständigkeit aus. Weißer oder klarer Kunststoff kann zudem für Umgebungslicht durchlässig sein, so dass Infrarotstrahlung aus der Umgebung in die Kammer treten kann und dort für die Aufzeichnung der Augenbewegung eingesetzt werden kann. Es kann somit auch eine Aufzeichnung der Augenbewegung mit sichtbarem Licht durchgeführt werden. Es können mit sichtbarem Licht deutlich detailreichere Bildinformation aufgenommen werden. Eine erhebliche Verbesserung der Erkennung feinster Strukturmerkmale führt zu einer höheren Genauigkeit der Messung der Augenbewegungen. Hierbei muss allerdings der Reflexionsbereich des Infrarotspiegels vergrößert werden. Mit bekannten Infrarotspiegeln wird Licht unterhalb von 700 nm Wellenlänge durchgelassen und oberhalb von dieser Schwelle reflektiert. Vorliegend ist die Grenze nach unten zu verschieben um mehr sichtbares Licht in die Kamera zu projizieren, wobei aber darauf geachtet werden muss, keine störenden Spiegelungen etwa des Auges selbst für den Probanden sichtbar zu machen.

Die raue Oberfläche kann das Material zudem eintrüben, so dass es nicht möglich ist, mit dem Auge Strukturen zu erkennen die außerhalb der Kammer liegen. Ist dies der Fall, so kann das Auge sich, wie für die Untersuchung gefordert, nicht fokussieren.

Die Abdeckung kann von dem Brillenkörper, vorzugsweise über eine magnetische Verbindung, eine Steckverbindung oder eine Clipsverbindung, bestimmungsgemäß trennbar sein. So kann die Abdeckung leicht vom Brillenkörper gelöst und wieder befestigt werden. Dies kann es ermöglichen, dass die Augenbewegung auch ohne Abdeckung in einem Freisichtverfahren gemessen wird. Durch das im Zuge des Abnehmens der Abdeckung reduzierte Gewicht ist die Vorrichtung im Freisichtmodus für manche Verfahren, etwa beim Video-Kopfimpulstest, leichter und damit vorteilhaft.

In konkreter Ausführung kann die Abdeckung an dem Brillenkörper aus einem von der Einblicköffnung freigegebenen Sichtfeld schwenkbar sein. Die Abdeckung ist in einem solchen Fall zwar immer fest mit dem Brillenkörper verbunden, das Sichtfeld kann über das Verschwenken der Abdeckung erweitert werden. Dies hat den Vorteil, dass die Abdeckung stets im Bereich der Vorrichtung behalten und nicht vollständig getrennt wird und verloren werden kann.

Die Abdeckung kann weiter Außenkanten aufweisen, welche sich bei bestimmungsgemäßer Verbindung mit dem Brillenkörper außerhalb des von der Einblicköffnung freigegebenen Sichtfelds befinden. Wenn im Sichtfeld des Auges keine scharfen Kanten vorliegen, entsteht ein unscharfer visueller Sinneseindruck, weil die Linsen des Auges nicht fokussiert sind. In diesem Falle ist es leichter, die Augenbewegung zu messen, weil die Augen nicht auf einen Punkt konzentriert sind und nicht bei einer Bewegung dem verfolgten Punkt nachschwenken.

Vorzugsweise kann die Einblicköffnung zwei Augenöffnungen umfassen und jeder Augenöffnung kann eine einsehbare, im Übrigen allerseits verschlossene Kammer mit einer, vorzugsweise abnehmbaren oder aus dem Sichtfeld verschwenkbaren, Abdeckung zugeordnet sein. Um die Bewegung nur eines Auges zu messen kann es vorteilhaft sein, dass die Einblicköffnung zwei Augenöffnungen aufweist. So kann nur eine Abdeckung entfernt werden, sodass zunächst ein Auge fokussieren kann und das Sichtfeld des anderen Auges weiterhin auf eine Kammer begrenzt bleibt. Dies ermöglicht eine getrennte Messung der beiden Augen unabhängig voneinander.

In Weiterbildung der Erfindung kann insbesondere auch vorgesehen sein, dass dem Brillenkörper ein Bewegungssensor, vorzugsweise ein Gyroskop oder ein mehrachsiger, vorzugsweise neunachsiger, Bewegungssensor zur Erfassung eines Auslenkungswinkels des Brillenkörpers zugeordnet ist.

Bei der Kalibrierung wird der Proband in diesem Fall aufgefordert, einen festen Punkt zu fixieren, wie etwa eine Markierung an einer gegenüberliegenden Wand oder einen Gegenstand. Sodann bewegt der Proband, gegebenenfalls vom Untersuchenden unterstützt, langsam den Kopf nach rechts, links, oben und unten durch, wobei sein Blick stets auf das Fixationsziel gerichtet bleibt. Die Auslenkung des Kopfes wird dabei von dem Bewegungssensor in Grad gemessen. Die Bewegung des jeweiligen Auges entgegen der Kopfbewegung wird dabei von den Kameras erfasst. Eine Analysesoftware kann dann die Änderung der Pupillenposition im Videobild mit der in Grad gemessenen Kopfbewegung korrelieren und so zukünftige Augenbewegungen in Grad angeben.

Das Messprinzip entspricht weitgehend der eigentlichen Kopfimpulstest-Messung. Es wird jedoch zum einen eine langsame Kopfbewegung ausgeführt, die auch bei Beeinträchtigung der zu diagnostizierenden Bogengänge von Patienten aufgrund der bewussten, nicht reflexartigen, visuellen Steuerung der Okulomotorik durchführbar ist, zum anderen wird von dem Bewegungssensor der statische Winkel im Raum gemessen, oder im einfacheren Fall eines reinen Gyroskops nur die Winkelgeschwindigkeit. Die Berechnung eines Auslenkungswinkels durch Integration der Winkelgeschwindigkeit ist auch bei Gyroskopen möglich, bevorzugt wird aufgrund der erheblich besseren Genauigkeit aber der mehrachsige Bewegungssensor, vorzugsweise mit neun Bewegungsachsen.

In konkreter Ausführung kann dem Brillenkörper ein, vorzugsweise längenverstellbares, Kopfband zur Befestigung an einem Kopf zugeordnet sein. Der Brillenkörper kann über ein längenverstellbares Band passgenau an die Kopfform angepasst werden. Eine passgenaue Anpassung des Kopfbands an den Kopf ermöglicht es, dass die Vorrichtung dicht an dem Gesicht des Probanden anliegt und keine Eindrücke von außen auf das Innere der Kammer zwischen Brillenkörper und Abdeckung eindringen kann.

Der wenigstens einen Einblicköffnung kann eine diese umgebende Gesichtsauflage, vorzugsweise aus Silikon oder Gummi, zugeordnet sein. Die Gesichtsauflage kann somit elastisch sein und damit die Anpassung der Vorrichtung an unterschiedliche Gesichtsformen möglich machen. Dies ist von besonderem Vorteil, da das Sichtfeld während der Messung mit Infrarotstrahlung möglichst auf die Kammer beschränkt sein soll.

Vorzugsweise kann dem Brillenkörper wenigstens eine Belüftungsöffnung außerhalb eines von der Einblicköffnung freigegebenen Sichtfelds zugeordnet sein. Es kann eine ausreichende Belüftung im inneren des Brillenkörpers über Belüftungsöffnungen gewährleistet sein, was sowohl das Gesicht des Probanden, als auch die Elektronik im Gehäuse betrifft. Vor allem bei sehr heißen Temperaturen kann die passgenau und eng anliegende Vorrichtung unangenehm beim Tragen sein. Dies kann verhindert werden, indem über die genannten Belüftungsöffnungen eine ausreichende Belüftung gewährleistet ist. Eine solche Belüftung ist nur möglich, da bei der vorliegenden Brille kein Lichtausschluss erforderlich ist. Bei Videobrillen, die auf dem Lichtausschlussprinzip basieren, ist eine Belüftung praktisch nicht durchführbar, da durch die Lüftungsschlitze immer auch Licht eindringt.

Dem Brillenkörper kann ein in Blickrichtung eines Benutzers gerichteter Richtlaser zugeordnet sein. Der Richtlaser projiziert mehrere Lichtpunkte an eine Wand, die in einem festen Winkel zueinander von dem Richtlaser abgestrahlt werden. Der Proband wird in einem Kalibrierschritt vom Untersuchenden aufgefordert, die Punkte abwechselnd zu fokussieren. Die Augen des Probanden werden dabei von den Kameras aufgezeichnet. Die Analyse der fixierten Punkte erlaubt es dem System, die Strecke zwischen den Fixationspunkten im Kamerabild mit dem bekannten Winkel zwischen den Laserpunkten zu korrelieren und so der Bewegung der Pupille im Bild einen Weg in Grad zuzuordnen.

In konkreter Ausführung kann der Brillenkörper und/oder die wenigstens eine Abdeckung aus einem thermoplastischen Kunststoff hergestellt sein, vorzugsweise aus Polymethylmethacrylat, Polyamid oder Polyacrylsäureester. Diese Kunststoffe haben vorteilhafte Materialeigenschaften, da sie thermisch verformbar sind. Aus diesen Kunststoffen können einfach und kostengünstig Brillenkörper oder Abdeckungen gefertigt werden, vorzugsweise über Spritzguss- oder 3D-Druck-Verfahren. Polymethylmethacrylat ist besonders vorteilhaft, da es nach der Verarbeitung durchsichtig ist und Infrarotstrahlung aus der Umgebung durchlässt.

Vorzugsweise kann der thermoplastische Kunststoff wenigstens eine raue Oberfläche aufweisen, wobei die raue Oberfläche vorzugsweise über ein Sandstrahlverfahren hergestellt ist. Ein klarer Kunststoff ist zunächst nicht undurchsichtig, somit kann die Oberfläche des Kunststoffes über ein Sandstrahlverfahren aufgeraut werden. Eine raue Oberfläche ist besonders vorteilhaft, da diese die sichtbare Strahlung diffus reflektiert und somit ein unscharfer optischer Sinneseindruck für den Träger der Vorrichtung entsteht, auf die er seine Augen nicht fokussieren kann.

Die vorstehend beschriebene Erfindung wird im Folgenden anhand eines Ausführungsbeispiels näher erläutert.

Es zeigen
- Figur 1: eine kopfgetragene Vorrichtung ohne Abdeckungen in perspektivischer Darstellung,
- Figur 2: die Vorrichtung gemäß Figur 1 in perspektivischer Schnittdarstellung,
- Figur 3: die Vorrichtung gemäß Figur 1 mit eingesetzten Abdeckungen in perspektivischer Darstellung, sowie
- Figur 4: die Vorrichtung gemäß Figur 3 in einer rückwärtigen Draufsicht auf die Einblicköffnungen.

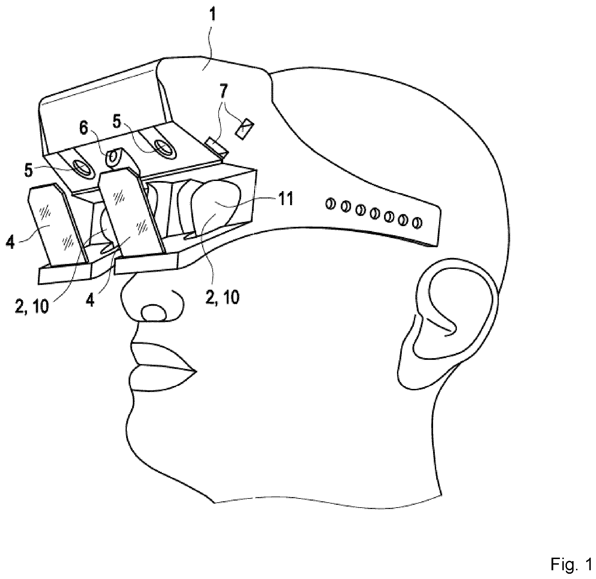

Figur 1 zeigt eine kopfgetragene Vorrichtung, welche einen Brillenkörper 1 umfasst. Der Brillenkörper 1 weist zwei Einblicköffnungen 2 auf, über die ein Proband in der gezeigten Konfiguration durch den Brillenkörper 1 hindurchsehen kann. Oberhalb der Einblicköffnungen 2 ist ein Gehäuseteil angeordnet, in dem zwei Infrarotkameras 5 angeordnet und abwärts auf zwei Infrarotspiegel 4 gerichtet sind. Die Infrarotspiegel 4 ermöglichen es, dass die Infrarotkameras 5 die Augen des Probanden durch die Einblicköffnungen 2 erfassen können, ohne aber ihrerseits von dem Probanden fixiert werden zu können. Die Infrarotspiegel 4 spiegeln lediglich Licht im infraroten Bereich, während das sichtbare Spektrum hindurchfällt, so dass sie für diesen Bereich klar wie Fensterglas und daher praktisch unsichtbar sind.

Mithilfe eines Richtlasers 6 kann in dem gezeigten Betriebszustand zunächst eine Kalibrierung vorgenommen werden. Der Proband wird aufgefordert, abwechselnd die Augen zu schließen und mit seinem offen verbleibenden Auge fünf von dem Richtlaser 6 an eine gegenüberliegende Wand projizierte Lichtpunkte zu fixieren und zwischen den fixierten Punkten zu wechseln. Mit den hierbei erfassten Kamerabildern und zugehörigen Daten, insbesondere die Winkeländerungen, die auf eine Längenänderung an der Wand und damit auf deren Entfernung zurückgerechnet werden können, kann eine Kalibrierung der Infrarotkameras 5 vorgenommen werden. Durch die Aufzeichnung der Augenbewegung während der Fixation eines bestimmten Blickziels, das zu einer Auslenkung der Augen aus der lotrechten Blickposition um einen bekannten Winkel führt, kann im aufgezeichneten Bild ermittelt werden, welche Distanz im Bild, gemessen in Pixeln, dem vorgegebenen Winkel entspricht. Damit können fortan Augenbewegungen in Grad oder Gradsekunden angegeben werden. Alternativ ist eine Kopfbewegung möglich, während der Proband seinen Blick auf einen festen Punkt fixiert hält.

An den seitlichen Bügeln kann ein Kopfband befestigt werden, um den Brillenkörper 1 in Position zu halten. Je nach durchgeführten Tests können ruckartige Kopfbewegungen vorgenommen werden, welche den Brillenkörper 1 verrutschen lassen, so dass die Infrarotkameras ihre Kalibrierung verlieren. ein ausreichend fester Sitz ist daher hilfreich. Belüftungsöffnungen an den Seiten des Brillenkörpers erlauben eine ausreichende Belüftung der oberhalb der Einblicköffnungen 2 eingebauten Technik.

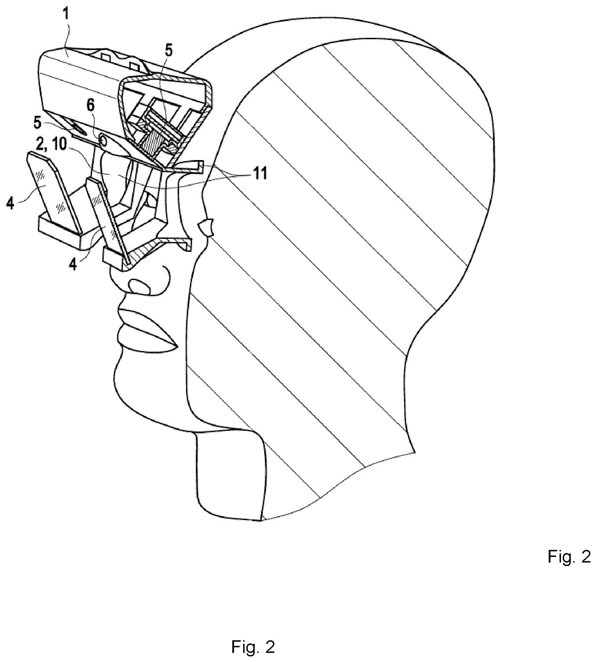

Diese ist in Figur 2 gezeigt. Neben den Infrarotkameras 5 befindet sich in einer Kavität oberhalb der Einblicköffnungen 2 die zugehörige Auswerteelektronik und Sensorik.

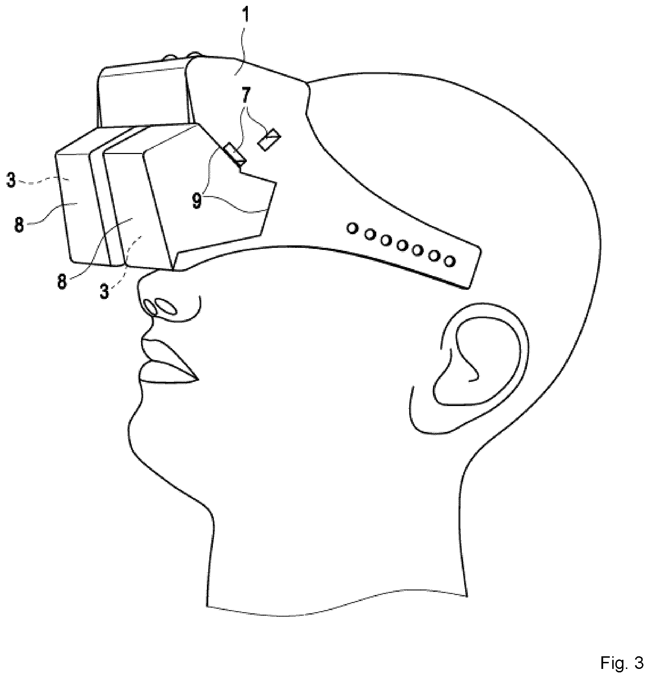

Figur 3 zeigt nunmehr die Vorrichtung gemäß Figur 1 mit zusätzlich aufgesetzten Abdeckungen 8. Diese sind aus einem transluzenten, trüben Material hergestellt, welches zwar erlaubt, dass Umgebungslicht durch das Material hindurchdringt und für gute Lichtverhältnisse im Inneren, der von den Abdeckungen 8 und dem Brillenkörper 1 eingeschlossenen Kammern 3 sorgt. Die Innenseiten der Abdeckungen 8 nehmen die darin mit eingeschlossenen Infrarotspiegel 4 auf, so dass weiterhin die Infrarotkameras 5 die Augen des Probanden erfassen können. Dadurch, dass an der Innenseite der Abdeckungen 8 alle Ecken und Kanten verrundet sind und die Innenflächen der Abdeckungen 8 damit kantenfrei ausgeführt sind, besteht für den Probanden kein Fixationspunkt, obwohl ausreichend Licht in den Abdeckungen 8 vorhanden wäre. Auch die Außenkanten 9 der Abdeckungen sind in der Kammer entweder außerhalb des Sichtfelds des Probanden oder anderweitig verdeckt. Wahlweise ist es möglich, auch nur eine Abdeckung 8 zu positionieren um nur ein Auge einer Freisichtanwendung, das andere einer Anwendung zu unterziehen, bei welcher die Fixation unterdrückt wird.

Eine solche Ausführung der Abdeckungen ist etwa durch eine additive Herstellung möglich. Dadurch, dass das Innere der Abdeckungen damit vollkommen diffus erscheint und keine Fixationspunkte gegeben sind, kann auf die bisher übliche, aber nie zufriedenstellend realisierbare Dunkelheit in den Kammern 3 verzichtet werden. Eine Beeinträchtigung des Eye Trackings durch die Reflexion der Beleuchtung in der Pupille kann nicht mehr stattfinden. Die Aufzeichnung des Augenbildes kann zudem mit Farbkameras im Bereich des sichtbaren Lichts erfolgen. Die damit deutlich detailreichere Bildinformation ermöglicht eine erhebliche Verbesserung der Erkennung feinster Strukturmerkmale und führt zu einer höheren Genauigkeit des Eye Trackings insbesondere der torsionalen Augenbewegung. Der bestimmungsgemäße Gebrauch des Gerätes bedingt nicht mehr die Abgabe von Energie auf den menschlichen Körper, da die Augenbeleuchtung mit Infrarotlicht entfällt. Dadurch wird das Risiko des Probanden erheblich minimiert, da eine hochdosierte Infrarotbestrahlung des Auges zur Erblindung führen kann.

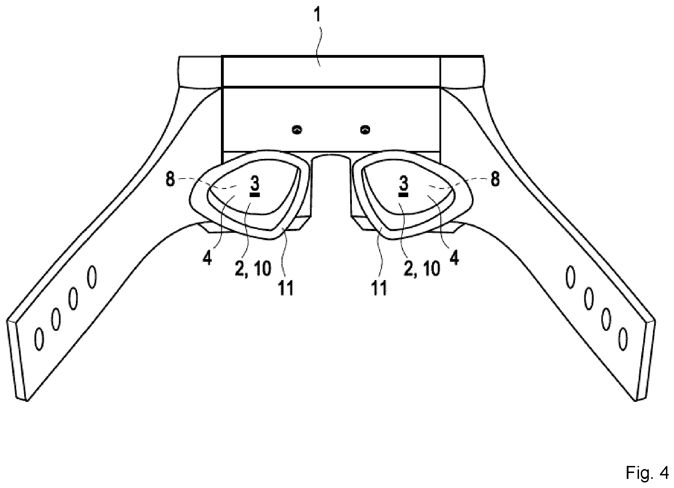

Schließlich zeigt Figur 4 eine Ansicht des Brillenkörpers 1 von einer Innenseite her. Durch die Augenöffnungen 10 hindurch kann vom Probanden, in die zwischen dem Brillenkörper 1 und der jeweiligen Abdeckung 8 gebildeten Kammern 3 blicken, wobei er zwar ein diffuses Bild hat und keinen Punkt findet, den er fixieren kann, dennoch aber durch die Umgebungsbeleuchtung Helligkeit wahrnimmt. Entsprechend können auch die Infrarotkameras 5 ein vollständiges Spektrum aufnehmen und müssen sich nicht auf den Infrarotbereich beschränken. Gesichtsauflagen 11 um die Augenöffnungen 10 herum sorgen für ein angenehmes Tragegefühl.

Vorstehend beschrieben ist somit eine kopfgetragene Vorrichtung zur Aufzeichnung einer Augenbewegung, welche die Nachteile des bekannten Standes der Technik im Hinblick auf die Fixation aufgrund unerwünschten Lichteinfalls behebt und die Erfassung einer torsionalen Augenbewegung verbessert.

### BEZUGSZEICHENLISTE

- 1: Brillenkörper
- 2: Einblicköffnung
- 3: Kammer
- 4: Infrarotspiegel
- 5: Infrarotkamera
- 6: Richtlaser
- 7: Belüftungsöffnung
- 8: Abdeckung
- 9: Außenkante
- 10: Augenöffnung
- 11: Gesichtsauflage

## Patentansprüche

1. Kopfgetragene Vorrichtung zur Aufzeichnung einer Augenbewegung, mit einem Brillenkörper (1), welcher gemeinsam mit einer Abdeckung (8) eine allseits geschlossene, jedoch über eine Einblicköffnung (2) einsehbare Kammer (3) ausbildet, wobei der Kammer (3) wenigstens ein im sichtbaren Spektrum durchsichtiger Infrarotspiegel (4) zur Reflexion von Infrarotlicht auf wenigstens eine oberhalb der Kammer (3) angeordnete Infrarotkamera (5) zugeordnet ist,
**dadurch gekennzeichnet, dass** der Brillenkörper (1) und die Abdeckung (8) aus einem trüben und transluzenten Material hergestellt ist und eine der Kammer (3) zugewandte, kantenfreie Innenfläche aufweist.

2. Vorrichtung gemäß Anspruch 1, **dadurch gekennzeichnet, dass** der Brillenkörper (1) und/oder die Abdeckung (8) aus einem Material aus einem weißen Kunststoff oder einem klaren Kunststoff mit angerauter Oberfläche hergestellt ist.

3. Vorrichtung gemäß Anspruch 1 oder 2, **dadurch gekennzeichnet, dass** die Abdeckung (8) von dem Brillenkörper (1), vorzugsweise über eine magnetische Verbindung, eine Steckverbindung oder eine Clipsverbindung, bestimmungsgemäß trennbar ist.

4. Vorrichtung gemäß Anspruch 1 oder 2, **dadurch gekennzeichnet, dass** die Abdeckung (8) an dem Brillenkörper (1) aus einem von der Einblicköffnung (2) freigegebenen Sichtfeld schwenkbar ist.

5. Vorrichtung gemäß einem der vorhergehenden Ansprüche, **dadurch gekennzeichnet, dass** die Abdeckung (8) Außenkanten (9) aufweist, welche sich bei bestimmungsgemäßer Verbindung mit dem Brillenkörper (1) außerhalb des von der Einblicköffnung (2) freigegebenen Sichtfelds befinden.

6. Vorrichtung gemäß einem der vorhergehenden Ansprüche, **dadurch gekennzeichnet, dass** die Einblicköffnung (2) zwei Augenöffnungen (10) umfasst und dass jeder Augenöffnung (10) eine einsehbare, im Übrigen allerseits verschlossene Kammer (3) mit einer, vorzugsweise abnehmbaren oder aus dem Sichtfeld verschwenkbaren, Abdeckung (8) zugeordnet ist.

7. Vorrichtung gemäß einem der vorhergehenden Ansprüche, **dadurch gekennzeichnet, dass** dem Brillenkörper (1) ein Bewegungssensor, vorzugsweise ein Gyroskop oder ein mehrachsiger, vorzugsweise neunachsiger, Bewegungssensor zur Erfassung eines Auslenkungswinkels des Brillenkörpers (1) zugeordnet ist.

8. Vorrichtung gemäß einem der vorhergehenden Ansprüche, **dadurch gekennzeichnet, dass** dem Brillenkörper (1) ein, vorzugsweise längenverstellbares, Kopfband zur Befestigung an einem Kopf zugeordnet ist.

9. Vorrichtung gemäß einem der vorhergehenden Ansprüche, **dadurch gekennzeichnet, dass** der Einblicköffnung (2) eine diese umgebende Gesichtsauflage(11), vorzugsweise aus Silikon oder Gummi, zugeordnet ist.

10. Vorrichtung gemäß einem der vorhergehenden Ansprüche, **dadurch gekennzeichnet, dass** dem Brillenkörper (1) wenigstens eine Belüftungsöffnung (7) außerhalb eines von der Einblicköffnung (2) freigegebenen Sichtfelds zugeordnet ist.

11. Vorrichtung gemäß einem der vorhergehenden Ansprüche, **dadurch gekennzeichnet, dass** dem Brillenkörper (1) ein in Blickrichtung eines Benutzers gerichteter Richtlaser (6) zugeordnet ist.

12. Vorrichtung gemäß einem der vorhergehenden Ansprüche, **dadurch gekennzeichnet, dass** der Brillenkörper (1) und/oder die wenigstens eine Abdeckung (8) aus einem thermoplastischen Kunststoff hergestellt ist, vorzugsweise aus Polymethylmethacrylat, Polyamid oder Polyacrylsäureester.

13. Vorrichtung gemäß Anspruch 12, **dadurch gekennzeichnet, dass** der thermoplastische Kunststoff wenigstens eine raue Oberfläche aufweist, wobei die raue Oberfläche vorzugsweise über ein Sandstrahlverfahren hergestellt ist.

## Claims

1. Head-mounted device for recording eye movement, comprising a spectacle frame (1) which, together with a cover (8), forms a chamber (3) that is closed on all sides but can be viewed through a viewing opening (2), wherein the chamber (3) is assigned at least one infrared mirror (4) which is transparent in the visible spectrum for reflecting infrared light onto at least one infrared camera (5) arranged above the chamber (3),
**characterised in that** the spectacle frame (1) and the cover (8) are made of an opaque and translucent material and have an edge-free inner surface facing the chamber (3).

2. Device according to claim 1, **characterised in that** the spectacle frame (1) and/or the cover (8) are made of a white plastic or a clear plastic with a roughened surface.

3. Device according to claim 1 or 2, **characterised in that** the cover (8) can be separated from the spectacle frame (1) in the intended manner, preferably by means of a magnetic connection, a plug connection or a clip connection.

4. Device according to claim 1 or 2, **characterised in that** the cover (8) can be pivoted on the spectacle frame (1) from a field of vision cleared by the viewing aperture (2).

5. Device according to one of the preceding claims, **characterised in that** the cover (8) has outer edges (9) which, when connected to the spectacle frame (1) as intended, are located outside the field of vision exposed by the viewing aperture (2).

6. Device according to one of the preceding claims, **characterised in that** the viewing aperture (2) comprises two eye openings (10) and that each eye opening (10) is assigned a viewable chamber (3) which is otherwise closed on all sides and has a cover (8) which is preferably removable or can be pivoted out of the field of view.

7. Device according to one of the preceding claims, **characterised in that** the spectacle frame (1) is assigned a motion sensor, preferably a gyroscope or a multi-axis, preferably nine-axis, motion sensor for detecting a deflection angle of the spectacle frame (1).

8. Device according to one of the preceding claims, **characterised in that** the spectacle frame (1) is assigned a headband, preferably adjustable in length, for attachment to a head.

9. Device according to one of the preceding claims, **characterised in that** the viewing aperture (2) is assigned a surrounding face rest (11), preferably made of silicone or rubber.

10. Device according to one of the preceding claims, **characterised in that** the spectacle frame (1) is assigned at least one ventilation opening (7) outside a field of vision exposed by the viewing opening (2).

11. Device according to one of the preceding claims, **characterised in that** the spectacle frame (1) is associated with a directional laser (6) directed in the user's line of sight.

12. Device according to one of the preceding claims, **characterised in that** the spectacle frame (1) and/or the at least one cover (8) is made of a thermoplastic material, preferably polymethyl methacrylate, polyamide or polyacrylic acid ester.

13. Device according to claim 12, **characterised in that** the thermoplastic material has at least one rough surface, wherein the rough surface is preferably produced by a sandblasting process.

## Revendications

1. Dispositif porté sur la tête pour enregistrer les mouvements oculaires, comprenant une monture de lunettes (1) qui, conjointement avec un cache (8), forme une chambre (3) fermée de tous côtés mais visible à travers une ouverture d'observation (2), la chambre (3) est associé au moins un miroir infrarouge (4) transparent dans le spectre visible pour réfléchir la lumière infrarouge vers au moins une caméra infrarouge (5) disposée au-dessus de la chambre (3),
**caractérisé en ce que** la monture de lunettes (1) et le cache (8) sont fabriqués dans un matériau opaque et translucide et présentent une surface intérieure sans arêtes tournée vers la chambre (3).

2. Dispositif selon la revendication 1, **caractérisé en ce que** la monture de lunettes (1) et/ou le cache (8) sont fabriqués dans un matériau en plastique blanc ou en plastique transparent à surface rugueuse.

3. Dispositif selon la revendication 1 ou 2, **caractérisé en ce que** le cache (8) peut être séparé de la monture de lunettes (1) de manière conforme à l'usage prévu, de préférence au moyen d'une connexion magnétique, d'une connexion enfichable ou d'une connexion à clip.

4. Dispositif selon la revendication 1 ou 2, **caractérisé en ce que** le cache (8) peut pivoter sur la monture (1) à partir d'un champ de vision dégagé par l'ouverture d'observation (2).

5. Dispositif selon l'une des revendications précédentes, **caractérisé en ce que** le cache (8) présente des bords extérieurs (9) qui, lorsqu'il est relié conformément à sa destination au corps de lunettes (1), se trouvent à l'extérieur du champ de vision dégagé par l'ouverture d'observation (2).

6. Dispositif selon l'une des revendications précédentes, **caractérisé en ce que** l'ouverture d'observation (2) comprend deux ouvertures oculaires (10) et **en ce qu'**à chaque ouverture oculaire (10) est associée une chambre (3) visible, par ailleurs fermée de tous côtés, avec un cache (8) de préférence amovible ou pivotant hors du champ de vision.

7. Dispositif selon l'une des revendications précédentes, **caractérisé en ce qu'**un capteur de mouvement, de préférence un gyroscope ou un capteur de mouvement multiaxial, de préférence à neuf axes, est associé à la monture de lunettes (1) pour détecter un angle de déviation de la monture de lunettes (1).

8. Dispositif selon l'une des revendications précédentes, **caractérisé en ce qu'**un bandeau, de préférence réglable en longueur, est associé à la monture de lunettes (1) pour la fixation sur la tête.

9. Dispositif selon l'une des revendications précédentes, **caractérisé en ce qu'**un appui-visage (11), de préférence en silicone ou en caoutchouc, est associé à l'ouverture d'observation (2) et l'entoure.

10. Dispositif selon l'une des revendications précédentes, **caractérisé en ce qu'**au moins une ouverture d'aération (7) est associée à la monture de lunettes (1) à l'extérieur d'un champ de vision dégagé par l'ouverture d'observation (2).

11. Dispositif selon l'une des revendications précédentes, **caractérisé en ce qu'**un laser directionnel (6) orienté dans la direction du regard d'un utilisateur est associé à la monture de lunettes (1).

12. Dispositif selon l'une des revendications précédentes, **caractérisé en ce que** la monture de lunettes (1) et/ou au moins un cache (8) sont fabriqués en matière thermoplastique, de préférence en polyméthacrylate de méthyle, en polyamide ou en ester d'acide polyacrylique.

13. Dispositif selon la revendication 12, **caractérisé en ce que** la matière thermoplastique présente au moins une surface rugueuse, la surface rugueuse étant de préférence réalisée par un procédé de sablage.
